# EUROPEAN PATENT SPECIFICATION

(11) **EP 0 232 085 B1**
(45) Date of publication and mention of the grant of the patent: **28.04.1993**
(21) Application number: 87300569.8
(22) Date of filing: 22.01.1987
(51) Int. Cl.: C12Q 1/68, C12Q 1/04, C07H 21/00

(54) **Campylobacter probe**
Campylobacter-Sonde
Sonde du campylobacter

(30) Priority: 22.01.1986 US 821393
(43) Date of publication of application: 12.08.1987
(73) Proprietor: AMOCO CORPORATION, Chicago Illinois 60680-0703 (US)
(72) Inventor: Rashtchian, Ayoub, Northboro, MA 01532 (US)
(74) Representative: Deans, Michael John Percy

(56) References cited:
- EP-A- 0 155 360
- WO-A-84/02721
- WO-A-86/04422

## Description

This invention relates to detecting bacteria belonging to the genus Campylobacter.

Detection of Campylobacter species is important in various medical contexts. Campylobacter jejuni and C. coli are the two most important species, causing diarrhea (Blaser et al., 1979, Ann. Intern, Med. 91:179), and enteritis (G.K. Morris et al., 1985, Manual of Medical Microbiology, Lennette et al., eds, American Society for Microbiology, Washington, D.C.) in humans. Other Campylobacter species have been implicated in causing disease in humans or animals, such as abortion, septicemia and proliferative ileitis. In addition, microorganisms resembling Campylobacter have been isolated from feces of homosexual men (Fennell et al., 1984 J. Infec. Dis. 149:58) and from gastric ulcer biopsies (Kasper et al., 1984 Infection. 12:179).

The presence of Campylobacter in clinical specimens (e.g., stool) is detected by culturing an appropriately prepared sample on microbiological media under conditions favorable for growth of these organisms. The resulting colonies are examined for morphological and biochemical characteristics, a process that typically is started 48 hours after aquisition of the sample and takes several days to complete.

Nucleic acid hybridizations (Nyguard, et al., 1964, J. Mol. Biol., 9:125) are performed in a variety of combinations (DNA/DNA, DNA/RNA, RNA/RNA), and can be performed in solution or on solid supports. Solid supports for hybridization assays are traditionally of nitrocellulose (Gilespie and Spiegelman, 1965 J. Mol. Biol. 12:829), or of paper with chemically reactive groups (Alwine et al., 1977, Proc. Nat. Acad. Sci. USA 74:4350). Development of more rapid and reliable formats for nucleic acid hybridization has allowed the use of this powerful technique in identification and detection of bacterial and viral pathogens. For example, Taber et al., EP-A-0114668 assigned to the same assignee and hereby incorporated by reference) discloses DNA probes for determining the presence of bacteria of the genus Salmonella in food; Falkow (US Patent 4,358,535) discloses a DNA probe for detecting enterotoxigenic E. coli in clinical specimens.

WO-A-8402721 discloses DNA probes which hybridize to Legionella and Trypanosoma rRNA respectively but not to rRNA of other widely different species. Also disclosed is a probe which hybridizes to Mollicutes rRNA and also to rRNA of various other bacteria, but not to mammalian cell rRNA.

This invention features the use of DNA probes in an assay to detect the presence of Campylobacter species in biological samples, such as feces, blood, or other body fluids or tissues. These probes can also be used to detect the presence of Campylobacter species in foods and biological samples or materials from animals. The assay is based on the complementary nature of these probes to the highly amplified ribosomal RNA (rRNA) sequences in Campylobacter cells.

The detection of Campylobacter in clinical specimens by the use of this invention employs one or more DNA probes which are complementary to rRNA of Campylobacter species. These DNA probes are specific for Campylobacter and do not hybridize to rRNA of any other bacteria. Each of these DNA probes is a DNA fragment which has been cloned from Campylobacter; or synthesized after determination of the nucleotide sequence of genes coding for rRNA in Campylobacter. Each of these probes hybridizes to most of the known Campylobacter species.

The invention features a DNA probe consisting essentially of a DNA sequence capable of hybridizing specifically to rRNA of a Campylobacter species but not to rRNA of a non-Campylobacter species. Generally this DNA probe is substantially complementary to RNA of the 5S, 16S, or 23S rRNA of Campylobacter ribosomes and in preferred embodiments has 15 or more bases and is substantially elementary (i.e., hybridizes to under normal hybridizing conditions) to a sequence of 15 or more bases of the RNA sequence (16S rRNA of C. jejuni): 
AAGCUUGCUA GCUUGCUAGA
AGUGGAUUAG UGGCGCACGG GUGAGUAAGG UAUAGUUAAU CUGCCCUACA
CAAGAGGACA ACAGUUGGAA ACGACUGCUA AUACUCUAUA CUCCUGCUUA
ACACAAGUUG ACUAGGGAAA GUUUUUCGGU GUAGGAUGAG ACUAUAUAGU
AUCAGCUAGU UGGUAAGGUA AUGGCUUACC AAGGCUAUGA CGCUUAACUG
GUCUGAGAGG AUGAUCAGUC ACACUGGAAC UGAGACACGG UCCAGACUCU
ACGGGAGGCA GCAGUAGGGA AUAUUGCGCA AUGGGGGAAA CCCUGACGCA
GCAACGCCGC GUGGAGGAUG ACACUUUUCG GAGCGUAAAC UCCUUUUCUU
AGGGAAGAAU UCUGACGGUA CCUAAGGAAU AAGCACCGGC UAACUCCGUG
CCAGCAGCCG CGGUAAUACG GAGGGUGCAA GCGUUACUCG GAAUCACUGG
GCGUAAAGGG CGCGUAGGCG GAUUAUCAAG UCUCAAGUGA AAUCUAAUGG
CUUAGCCAUU AAACUGCUUG GGAAACUGAU AGUCUAGAGU GCAGGGAGAG
GCAGAUGGAU UGGUGGUGUA GGGUAAAUCC UAGAUAUCAC AGAUACAUUG
CGAGGCGAUC UCUGGAUCCA UCGAGCCUA
or 15 or more bases of the sequence

ACCAAGAAUA CCCAUUGCGA AGGCGAUCUG CUGGAACUCA ACUGACGCUA
AGGCGCGAAA GCGUCCCCAG CAAACAGGAU UAAGAUACCC UUGUAGUCCA
CGCCCUAAAA CGACGUACAC UAGUUAUUCC CCUGCUAGUC AUCUCUGUAU
UGUCGCUAAC GCAUUAAGUG UACCGCCUAG GGUGUACGGU CGCAAGAUUA
AUUUCCGCAA CGAGCGCACC CACGUAUUUG UUGCUAACGG UUCGGACCGA
GACACUCUAA AUAGGCUGCC UUCGUAAGGA GGAGGAAGGU GUGGACGACG
UCAAGUCAUC AUGGCCCUUA UGCCCAAGGC GACACACGUG CUACAAUGGC
AUAUACAAUG AGACGCAAUA CCGCGAGGUU GGGCAAUCUA UAAAUAUGUC
CGGUUCGGAU UGUUCUCUGC AACUCGAGAG CAUGAAGCCG GAAUCGCUAG
UAAUCGUGGA UCAGCCAUGC UACGGUGAAU ACGUUCCCGG GUCUUGGAAC
UCACCGCCCG UCACACCAUG GGAGUUGAUU UCACUCGAAG CCGGAAUACU

AAACUAGUUA CCGUCCACAG UGGAAUCACC GGCGCUGGGG UGAAGUCGUA
ACAAGGUAAC CGUAGGAGAA CCUGCGGUCG GAUCACCUCC U.

In the preferred embodiments the DNA probe is labelled either radioactively, for example with ³²P, or nonradio- actively with a compound capable of being detected or of selectively binding to an indicator to form a detectable complex. Examples of such complexes include compounds such as avidin and biotin, antigens and antibodies, and enzymes and their corresponding substrates. Other nonisotopic labels include fluorescent compounds, electron dense compounds, acridine esters and luminescent lanthanides.

The probes of the invention are used for detecting the presence of Campylobacter in a sample, by contacting this probe with bacteria in the sample, under conditions that will allow the probe to hybridize to rRNA of any Campylobacter species present in the sample, and thus form a hybrid nucleic acid complex. The hybrid complex is detected and is an indication of the presence of Campylobacter in the sample.

The detection of Campylobacter using the DNA probes described below gives rapid and accurate results, allowing clinical laboratories to inform physicians of the presence or absence of Campylobacter in specimens in a short period of time. Furthermore, the test depends on the nucleic acid content of bacteria rather than other variable biological properties. This allows the detection of biochemically atypical as well ash typical strains of Campylobacter. In addition, since the assay does not necessarily require viable cells for detection, the need for use of transport media for transportation of specimens to the laboratory is eliminated.

Other features and advantages of the invention will be apparent from the following description of the preferred embodiments and from the claims.

### Description of the Preferred Embodiments

The drawings will first briefly be described.

### Drawings

Figure 1 is the nucleotide sequence of two portions of the 16S rRNA from C. jejuni.

Figure 2 is the DNA sequence of oligonucleotides AR196, AR197, AR327, AR328, and AR329.

### Structure

### Ribosomal RNA (rRNA)

Ribosomes consist of a complex mixture of proteins and RNA, and are responsible for translation of the genetic material in all organisms. There are three distinct ribosomal RNA molecules in bacteria (5S, 16S, 23S). Most bacteria have multiple copies of rRNA genes (Noller, 1984, Ann. Rev. Biochem. 53:119), and each bacterial cell contains approximately 1.2 x 10⁴ ribosomes, and therefore at least 1.2 x 10⁴ molecules of each rRNA. Other genes in bacteria exist as 1-2 copies per cell and their mRNA products are not stable, and are not transcribed at all times. Consequently, detection of rRNA by hybridization would be approximately 10⁴ - fold more sensitive than hybridization to DNA of other genes.

### Probes

The probes may be derived from the genomic DNA or ribosomal RNA of a Campylobacter cell or may be synthesized in vitro. These probes are essentially DNA seqences complementary to the rRNA sequences in Campylobacter. The probes need only be of 15 or more bases in length for their successful use in detection assays. These probes are generally labelled in any way that will enable their detection; for example isotopically or non-isotopically, as described by Mock et al. EP-A-0212951

### Campylobacter Strains

The genus Campylobacter has been described by R. M. Smibert in Bergey's Manual of Systematic Bacteriology (9th ed. 1984, Vol. 1, Williams and Wilkins).

Strains of Campylobacter, especially C. jejuni and C. coli, that can be used in the screening procedure described below are available from numerous sources, particularly from major medical centers. For example, well-characterized C. jejuni and C. coli strains are available from the University of Massachusetts Medical Center in Worcester, Massachusetts c/o Gary Doern. C. jejuni and C. coli strains are also available from Scott Laboratories, Inc., Fiskville, Rhode Island. Other sources of clinical isolates include the Veterans Administration Medical Center, Denver, Colorado (M.J. Blaser), Vermont Department of Public Health, and the American Culture Collection (ATCC), Rockville, MD.

### Non-Campylobacter Strains

Generally these are strains normally present in samples to be tested for the presence of Campylobacter and include: Escherichia coli, Enterobacter cloacae, Enterobacter agglomii, Enterobacter gergoviae, Citrobacter ferundii, Citrobacter diversus-levihea, Klebsiella pneumoniae, Klebsiella oxytoca, Proteus mirabilis, Proteaus morganii, Proteus rettgeri, Salmonella enteritidis ,Salmonella serotype Cl, Salmonella typhimurium, Serratia liquefaciens, Serratia marcescens, Shigella flexneri, Shigella sonneii, Shigella byoydii, Shigella dysenteriae, Vibrio parahaemolyticus, and Yersinia enterolitica.

### Methods

### Isolation of Probes

A genomic library of C. jejuni DNA is constructed in a suitable plasmid or phage vector. A number of suitable vectors are available and have been described in Maniatis et al. (1982) Molecular Cloning, Cold Spring Harbor Laboratory. One such vector is pUN121 (Nilsson et al., 1983, Nucleic Acids Research 11:8019). The DNA from C. jejuni N941 (ATCC 39983) is digested with a restriction endonuclease, e.g. HindIII, and ligated into an appropriate site of a suitable vector; using the general method described in Cohen et al. (1973, Proc. Nat'l Acad. Sci. 70:3240). This ligation mix is then used to transform competent E. coli cells, such as strain HB101. The transformants are selected on the basis of a suitable marker. The colonies of transformants are transferred to nitrocellulose membranes, and after lysis on the membrane filter are screened in hybridization experiments using ³²P-end labeled ribosomal RNA (rRNA) from Campylobacter (prepared according to conventional methods). From results of such hybridization experiments the colonies which contain recombinant plasmids carrying the DNA sequences from C. jejuni coding for rRNA can be identified. These plasmids, for example pAR140 (see below), are then characterized using standard restriction endonuclease analysis and hybridization (Maniatis et at., id.). After identification of DNA fragments coding for individual rRNA's (i.e. 16S, 23S, and 5S) by hybridization, the nucleotide sequence of their DNA (see below) is determined by standard methods (Maxam et al., 1980 Methods Enz. 65:499; Sanger et al., 1977, Proc. Nat. Acad. Sci. 74:5463). The nucleotide sequence is then compared to other published rRNA sequences and the regions of homology and non-homology are determined. The regions which are not homologous to other published sequences are subcloned or synthesized using automatic oligonucleotide synthesizers. These unique Campylobacter DNA fragments are labeled with ³²P and used as probes to be tested against both Campylobacter and non-Campylobacter strains. If desired the probe sequence may be incorporated into a vector and the resulting vector used as a probe; the vector should not have homology to either Campylobacter or non-Campylobacter species which may be present in the sample being tested. Probe sequences which are specific to Campylobacter strains are described below.

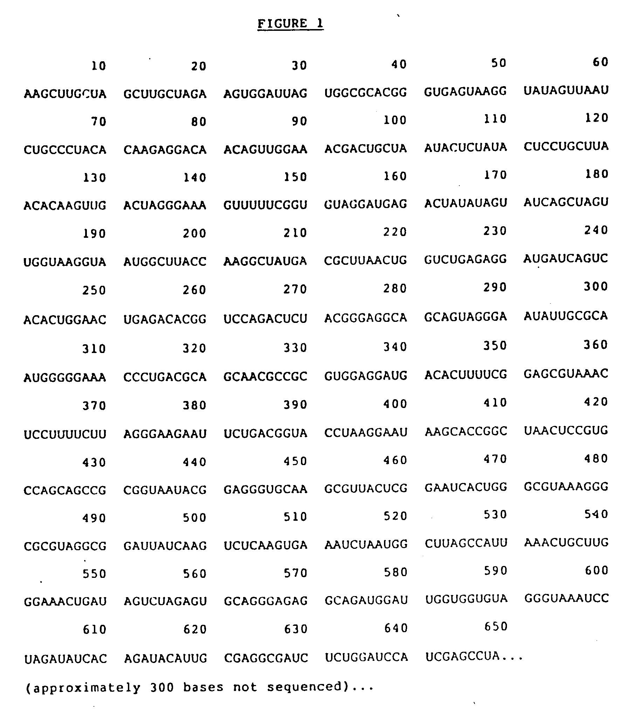

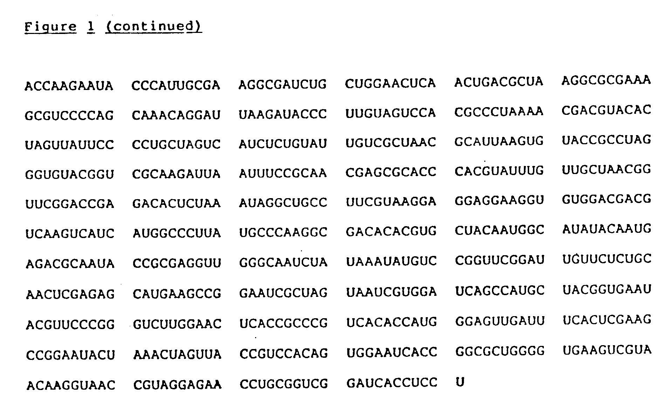

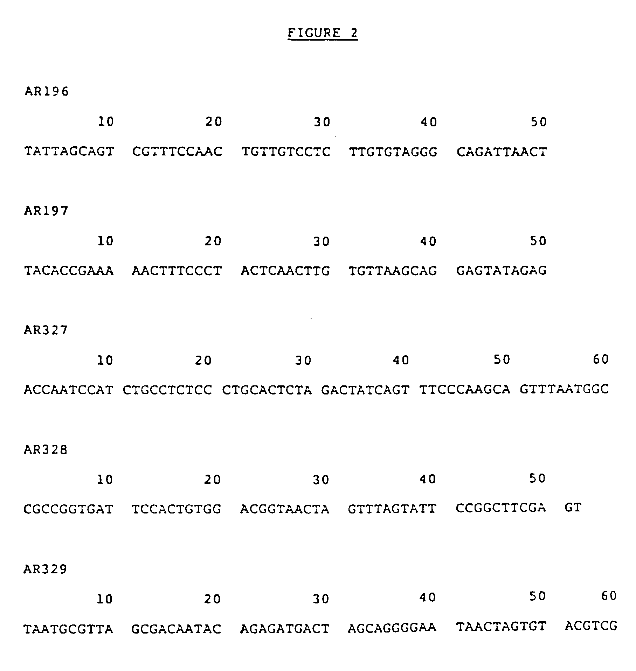

Figure 1 gives the nucleotide sequence of two portions of the 16S rRNA deduced from the sequence of the DNA in the clone, pAR140. Comparison of this sequence to the nucleotide sequence of rRNA genes from E. coli (Brosius et al., 1981 J. Mol. Biol. 148:107) shows considerable homology between the two genes. However, several regions of the molecules do differ and include the sequence between nucleotides 53 and 153 in the first sequence shown in Fig. 1. These regions of non-homology to E. coli were compared with other published rRNA sequences of bacteria and eucaryotes (Nelles et al., 1984 Nucl. Acid Res. 12:8749), and with the entire nucleic acid data bank of Scisoft, Inc. using the Microgenie™ computer program to look for significant homology. No sequence homology was found between the sequences from nucleotide 53 to 153 in the first sequence in Figure 1 and the sequences present in the data banks. Two synthetic oligonucleotides were derived from this sequence, synthesized, and then tested against a variety of Campylobacter and non-Campylobacter strains in hybridization assays. By way of example, Figure 2 shows the nucleotide sequence of two synthetic oligonucleotides, AR196 and AR197. These probes have been shown to be specific for Campylobacter strains and do not hybridize to DNA or RNA from non-Campylobacter bacteria. Similarly three synthetic oligonucleotides have been synthesized from the second part of the sequence in Fig. 1, these are shown in Fig. 2 as AR327-329. These too are specific to the rRNA of Campylobacter species.

In addition to the sequences shown in Figs. 1 and 2, there are other regions which are likely to be useful as probes. Although in the example above only the sequences of the 16S rRNA were discussed, the same analysis can be used for 23S rRNA and 5S rRNA. Using a different strategy (see below) we identified a region of the gene coding for 23S rRNA which appears to be specific for Campylobacter. This region was identified by restriction digestion of the DNA fragment coding for 232 rRNA and Southern blot hybridization to end labeled rRNA from E. coli and Campylobacter. In this manner a 600bp fragment within the 23S rRNA gene was found to be non- homologous to rRNA of E. coli. Similar analysis of this fragment with rRNA from other bacteria can establish suitability of this DNA fragment as a probe.

### Alternative Methods of Probe Isolation

In addition to the methods described above, there are other procedures that can be applied to isolate and identify DNA sequences complementary to rRNA of Campylobacter which are not homologous to the rRNA of other organisms. One of these methods is the use of the enzyme reverse transcriptase. This enzyme uses RNA as a template and transcribes a DNA strand (cDNA) which is complementary to the RNA template. Using this enzyme cDNA can be synthesized from rRNA of Campylobacter. Isolation of the Campylobacter-specific cDNA is performed in one of two ways, as follows:
(a) The cDNA are hybridized in solution to the rRNA of other bacterial or eucaryotic organisms. The cDNA which is homologous to the non-Campylobacter organisms will form double stranded DNA/RNA hybrids. These hybrids can then be separated from the non-hybridized fractions of the cDNA by hydroxyapatite chromatography. The methods for hydroxyapatite chromatography have been described by Bernardi (1969, Biochem. Biophys. Acta 174:423). The cDNA which remains single stranded can be used as a Campylobacter-specific probe or can be subcloned and isolated. This method can be repeated several times with distinct rRNA's from various sources.
(b) The cDNA from Campylobacter rRNA is hybridized to total genomic DNA or to cloned rRNA genes from other bacteria which have been labeled with biotin. After hybridization, the DNA is passed through one or more columns with bound avidin, which adsorbs all of the biotinylated DNA and thus any DNA which has hybridized to the biotinylated DNA. The DNA which does not hybridize, and thus has little or no homology to other bacterial DNA, is not retained by the column and, therefore, can be collected and used as a probe or subcloned and isolated.

### Examination of Stool Specimens

The specificity of the synthetic oligonucleotide probes for Campylobacter of Fig. 2 has been determined by hybridization experiments. It has been shown that these oligonucleotides hybridize specifically to the Campylobacter strains listed in Table 1, but not with non-Campylobacter strains.

**Table 1**

| CAMPYLOBACTER STRAINS TESTED WITH DNA PROBES | | |
|---|---|---|
| Species | Number of strains tested | Source |
| C. jejuni | 99 | a |
| C. coli | 15 | a |
| C. fetus | 8 | a |
| C. laridis 35221 | 1 | ATCC^{b} |
| C. fetus subsp. venerialis 19438 | 1 | ATCC^{b} |
| C. hyointestinalis 35217 | 1 | ATCC^{b} |

| | | |
|---|---|---|
| a. These strains were obtained from Gary Doern, University of Massachusetts Medical Center, M.J. Blaser, VA Medical Center, Denver, CO, and Tanya Sanderos, Vermont Department of Health, Burlington, VT. | | |
| b. American Type Culture Collection, Rockville, MD. | | |

The lack of hybridization to the bacteria normally present in stool specimens was shown as follows: Stool specimens from ten different healthy humans were screened with each of the DNA probes. For these experiments several different enrichment methods, as well as direct testing, were utilized. Direct testing of stool entailed spotting specimens onto nitrocellulose membrane filters and, after lysis of cells and fixing of the released DNA to the filters, they were hybridized to ³²P-labeled DNA probes. Enrichment methods entailed: (a) inoculation of stool specimens onto blood agar plates, and incubation under aerobic, microaerophilic or anaerobic conditions; or (b) inoculation of Campy-BAP plates and incubation under microaerophilic or aerobic conditions; or (c) inoculation of stool specimens in L-broth and incubation at 37°C with shaking. After enrichment the resulting bacteria were tested as for directly tested stool. No hybridization to the Campylobacter-specific DNA probes was observed. Only when these specimens were artificially inoculated with Campylobacter was hybridization to DNA probes observed.

### Use of Probes

Several methods and formats can be used for detection of Campylobacter species with DNA probes complementary to rRNA. The parameters and kinetics of DNA hybridization are well known in the art (D.E. Kennell, 1971, Progress in Nucleic Acid Research and Molecular Biology, Vol. 11, Academic Press, and Meinkoth et al., 1984 Anal. Biochem, 138:267), and those skilled in the art will recognize that it is possible to adjust hybridization conditions such as temperature and ionic strength, to provide different levels of selectivity and sensitivity.

Hybridizations ordinarily are performed in solution or on solid supports.

### (a) Solution Hybridization

The specimen and the probe DNA are mixed in solution and are allowed to hybridize. After hybridization, the single stranded and double stranded nucleic acids can be separated and quantitated by a variety of methods. For example: Nygaard et al. (1964 J. Mol. Biol. 9:125) described a method for quantitation of DNA/RNA hybrids after hybridization, by filtration through a nitrocellulose membrane. Another method involves the hydrolysis of single stranded nucleic acids with nuclease S1, followed by precipitation of non-hydrolyzed nucleic acids with acid (e.g. trichloroacetic acid). Double stranded hybrids formed in solution hybridization can also be detected by hydroxyapatite chromatography (Bernardi, 1971, Methods in Enzymology, 21D:95,) or by a centrifugation method as described by Brenner et al. (1969 Anal. Biochem. 28:447).

Another assay procedure involves the hybridization of rRNA from the sample to the complementary DNA probe in solution, followed by detection of the DNA/RNA hybrids using an antibody specific to DNA/RNA hybrids. Such antibodies have been described (Alcover et al., 1982 Chromosoma (Berl.) 87:263). These antibodies can be used in a variety of ways; for example the antibody can be conjugated to a solid phase (tubes, beads, etc.) or the DNA/RNA specific antibody can be fluorscinated and detected by a second antibody specific for fluorsceine, which has been conjugated to a solid phase; or the DNA/RNA antibody can be used in solution followed by the addition of protein A which has been immobilized on a solid phase (O'Keefe et al. 1980, J. Biol. Chem. 255:561) . Other methods which can be employed for use of antibodies are known in the art (Van Vunalis et al., 1985, Methods of Enzymology Vol. 70 Kicka. Ligand-binder assays, Labels and analytical strategies, Marcel Dekker, Inc. N.Y.).

### (b) Solid Phase Hybridization

One of the nucleic acids participating in hybridization is immobilized on a solid support. Usually the nucleic acid from the test sample is the immobilized species and is single stranded. A variety of solid supports are available and include nitrocellulose, nylon or derivatized papers. An example of such solid-phase hybridization has been disclosed by Taber and Fitts, EP-A-0 114 668 and by Rashtchian et al., EP-A-0 208 772 both assigned to the same assignee. A variety of ways by which RNA can be immobilized on the solid support are known in the art (Maniatis id.; Alwine et al., 1977, Proc. Nat. Acad. Sci., 74:4350; and Thomas, 1980 Proc. Nat. Acad. Sci., 77:5201).

A variation of the traditional solid phase hybridization is sandwich hybridization (Ranki et al. 1983, Gene 21:77). Originally, sandwich hybridizations were performed with nitrocellulose as solid support. However, other supports such as plastic, or Sepharose can also be used (Polsky-Cynkin et al., 1985 Clinical Chemistry, in press).

Another method that can be used is the immobilization of the DNA probe and hybridization of this with the released and purified rRNA from a sample. The presence of resulting rRNA/DNA hybrids on the filter can then be detected using the fluorscinated RNA/DNA specific antibodies discussed above.

In a quantitative assay, the DNA probes AR196 and AR197, shown in Fig. 2, were non-isotopically labelled with biotin EP-A-0 212 951 assigned to the same assignee) and used to probe cell lysates of C. jejuni and E. coli. Any resulting DNA/RNA hybrids were bound to plastic tubes using antibody to DNA/RNA hybrids. (The anti DNA/RNA antibody was prepared as described by Kitagawa et al. (1982, Mol. Immuno 9:413) and immobilized on the surface of a plastic tube by the method of Parsons (1973, Methods in Enzymol., 73:224).) The amount of biotin bound to the tubes was then estimated from the absorbance resulting after treatment of the tubes with avidin. The results are shown in the table below

| Type of Bacteria | Number of Cells | Absorbance |
|---|---|---|
| C. jejuni | 1 x 10⁸ | 1.75 |
| | 1 x 10⁷ | 0.38 |
| Escherichia coli | 5 x 10⁸ | 0.026 |

These results show that the probes AR196 and AR197 do not hybridize significantly to E. coli cell extracts, but do to those of C. jejuni.

### Other Embodiments

Other embodiments are within the following claims. The number of probes required depends in part on the sensitivity required in a given application and the tolerance for false negative results. In general one or more probes may be used.

Other strains of Campylobacter may be used in the initial cloning experiments as a source of DNA; as can other non-Campylobacter strains be used for subsequent probe testing.

## Claims

1. A DNA probe comprising 8 DNA sequence characterised in that the DNA sequence is capable of hybridizing specifically to rRNA of a Campylobacter species, and not capable of hybridizing specifically to rRNA of any non-Campylobacter species of bacteria.

2. A DNA probe as claimed in claim 1 wherein said rRNA comprises one of the 5S, 16S, or 23S subunit of ribosomes of said Campylobacter species.

3. A DNA probe as claimed in claim 1 or 2, wherein said probe has 15 or more bases and is substantially complementary to a sequence of 15 or more bases of the sequence 
AAGCUUGCUA GCUUGCUAGA
AGUGGAUUAG UGGCGCACGG GUGAGUAAGG UAUAGUUAAU CUGCCCUACA
CAAGAGGACA ACAGUUGGAA ACGACUGCUA AUACUCUAUA CUCCUGCUUA
ACACAAGUUG ACUAGGGAAA GUUUUUCGGU GUAGGAUGAG ACUAUAUAGU
AUCAGCUAGU UGGUAAGGUA AUGGCUUACC AAGGCUAUGA CGCUUAACUG
GUCUGAGAGG AUGAUCAGUC ACACUGGAAC UGAGACACGG UCCAGACUCU
ACGGGAGGCA GCAGUAGGGA AUAUUGCGCA AUGGGGGAAA CCCUGACGCA
GCAACGCCGC GUGGAGGAUG ACACUUUUCG GAGCGUAAAC UCCUUUUCUU
AGGGAAGAAU UCUGACGGUA CCUAAGGAAU AAGCACCGGC UAACUCCGUG
CCAGCAGCCG CGGUAAUACG GAGGGUGCAA GCGUUACUCG GAAUCACUGG
GCGUAAAGGG CGCGUAGGCG GAUUAUCAAG UCUCAAGUGA AAUCUAAUGG
CUUAGCCAUU AAACUGCUUG GGAAACUGAU AGUCUAGAGU GCAGGGAGAG
GCAGAUGGAU UGGUGGUGUA GGGUAAAUCC UAGAUAUCAC AGAUACAUUG
CGAGGCGAUC UCUGGAUCCA UCGAGCCUA
 orto a sequence of 15 or more bases of the sequence

ACCAAGAAUA CCCAUUGCGA
AGGCGAUCUG CUGGAACUCA ACUGACGCUA AGGCGCGAAA GCGUCCCCAG
CAAACAGGAU UAAGAUACCC UUGUAGUCCA CGCCCUAAAA CGACGUACAC
UAGUUAUUCC CCUGCUAGUC AUCUCUGUAU UGUCGCUAAC GCAUUAAGUG
UACCGCCUAG GGUGUACGGU CGCAAGAUUA AUUUCCGCAA CGAGCGCACC
CACGUAUUUG UUGCUAACGG UUCGGACCGA GACACUCUAA AUAGGCUGCC
UUCGUAAGGA GGAGGAAGGU GUGGACGACG UCAAGUCAUC AUGGCCCUUA
UGCCCAAGGC GACACACGUG CUACAAUGGC AUAUACAAUG AGACGCAAUA
CCGCGAGGUU GGGCAAUCUA UAAAUAUGUC CGGUUCGGAU UGUUCUCUGC
AACUCGAGAG CAUGAAGCCG GAAUCGCUAG UAAUCGUGGA UCAGCCAUGC
UACGGUGAAU ACGUUCCCGG GUCUUGGAAC UCACCGCCCG UCACACCAUG
GGAGUUGAUU UCACUCGAAG CCGGAAUACU AAACUAGUUA CCGUCCACAG
UGGAAUCACC GGCGCUGGGG UGAAGUCGUA ACAAGGUAAC CGUAGGAGAA
CCUGCGGUCG GAUCACCUCC U.

4. A DNA probe as claimed in any of claims 1 to 3 wherein said Campylobacter
species is one of C. jejuni, C. fetus subsp. jejuni, C. coli, Vibrio coli, C. fetus, C. fetus subsp. fetus, C. fetus subsp. intestinalis, C. fecalis, or C. hyointestinalis.

5. A DNA probe as claimed in any of claims 1 to 4 wherein said probe is labelled.

6. A method of detecting the presence of Campylobacter in a sample comprising:
providing at least one DNA probe that is capable of Selectively hybridizing specifically to the rRNA of a Campylobacter species but not to the rRNA of a species that does not belong to the genus Campylobacter,
contacting said DNA probe with bacteria in said sample under conditions that allow said probe to hybridize to rRNA of said Campylobacter species in said sample to form hybrid nucleic acid complexes, and
detecting said hybrid complexes as an indication of Campylobacter in said sample.

7. A method as claimed in claim 6 wherein said probe is derived from the 5S, 16S, or 23S subunits of Campylobacter ribosomal RNA.

8. A method as claimed in Claim 6 or 7 wherein said probe has 15 or more bases and is substantially complementary to a sequence of 15 or more bases of the sequence: 
AAGCUUGCUA GCUUGCUAGA
AGUGGAUUAG UGGCGCACGG GUGAGUAAGG UAUAGUUAAU CUGCCCUACA
CAAGAGGACA ACAGUUGGAA ACGACUGCUA AUACUCUAUA CUCCUGCUUA
ACACAAGUUG ACUAGGGAAA GUUUUUCGGU GUAGGAUGAG ACUAUAUAGU
AUCAGCUAGU UGGUAAGGUA AUGGCUUACC AAGGCUAUGA CGCUUAACUG
GUCUGAGAGG AUGAUCAGUC ACACUGGAAC UGAGACACGG UCCAGACUCU
ACGGGAGGCA GCAGUAGGGA AUAUUGCGCA AUGGGGGAAA CCCUGACGCA
GCAACGCCGC GUGGAGGAUG ACACUUUUCG GAGCGUAAAC UCCUUUUCUU
AGGGAAGAAU UCUGACGGUA CCUAAGGAAU AAGCACCGGC UAACUCCGUG
CCAGCAGCCG CGGUAAUACG GAGGGUGCAA GCGUUACUCG GAAUCACUGG
GCGUAAAGGG CGCGUAGGCG GAUUAUCAAG UCUCAAGUGA AAUCUAAUGG
CUUAGCCAUU AAACUGCUUG GGAAACUGAU AGUCUAGAGU GCAGGGAGAG
GCAGAUGGAU UGGUGGUGUA GGGUAAAUCC UAGAUAUCAC AGAUACAUUG
CGAGGCGAUC UCUGGAUCCA UCGAGCCUA
 or to a sequence of 15 or more bases of the sequence

ACCAAGAAUA CCCAUUGCGA
AGGCGAUCUG CUGGAACUCA ACUGACGCUA AGGCGCGAAA GCGUCCCCAG
CAAACAGGAU UAAGAUACCC UUGUAGUCCA CGCCCUAAAA CGACGUACAC
UAGUUAUUCC CCUGCUAGUC AUCUCUGUAU UGUCGCUAAC GCAUUAAGUG
UACCGCCUAG GGUGUACGGU CGCAAGAUUA AUUUCCGCAA CGAGCGCACC
CACGUAUUUG UUGCUAACGG UUCGGACCGA GACACUCUAA AUAGGCUGCC
UUCGUAAGGA GGAGGAAGGU GUGGACGACG UCAAGUCAUC AUGGCCCUUA
UGCCCAAGGC GACACACGUG CUACAAUGGC AUAUACAAUG AGACGCAAUA
CCGCGAGGUU GGGCAAUCUA UAAAUAUGUC CGGUUCGGAU UGUUCUCUGC
AACUCGAGAG CAUGAAGCCG GAAUCGCUAG UAAUCGUGGA UCAGCCAUGC
UACGGUGAAU ACGUUCCCGG GUCUUGGAAC UCACCGCCCG UCACACCAUG
GGAGUUGAUU UCACUCGAAG CCGGAAUACU AAACUAGUUA CCGUCCACAG
UGGAAUCACC GGCGCUGGGG UGAAGUCGUA ACAAGGUAAC CGUAGGAGAA
CCUGCGGUCG GAUCACCUCC U.

9. A method as claimed in any of claims 6 to 8 wherein said Campylobacter species is one of C. jejuni, C. fetus subsp. jejuni, C. coli, Vibrio coli, C. fetus, C. fetus subsp. fetus, C. fetus subsp. intestinalis, C. fecalis, or C. hyointestinalis.

10. A method as claimed in any of claims 6 to 9 wherein said sample is a gastroenteric sample, a stool sample or a food sample.

## Patentansprüche

1. DNS-Testmedium mit einer DNS-Folge,
dadurch gekennzeichnet, daß die DNS-Folge zu rRNS einer Campylobacter-Spezies spezifisch hybridisieren kann und zu rRNS irgendeiner Nicht-Campylobacter-Spezies von Bakterien nicht spezifisch hybridisieren kann.

2. DNS-Testmedium nach Anspruch 1,
dadurch gekennzeichnet, daß die rRNS eine oder mehr der 5S-, 16S- oder 23S-Untereinheiten von Ribosomen der Campylobacter-Spezies enthält.

3. DNS-Testmedium nach Anspruch 1 oder 2,
dadurch gekennzeichnet, daß das Testmedium 15 oder mehr Basen aufweist und im wesentlichen komplementär ist zu einer Folge von 15 oder mehr Basen der Folge 
AAGCUUGCUA GCUUGCUAGA
AGUGGAUUAG UGGCGCACGG GUGAGUAAGG UAUAGUUAAU CUGCCCUACA
CAAGAGGACA ACAGUUGGAA ACGACUGCUA AUACUCUAUA CUCCUGCUUA
ACACAAGUUG ACUAGGAAA GUUUUCGGU GUAGGAUGAG ACUAUAUAGU
AUCAGCUAGU UGGUAAGGUA AUGGCUUACC AAGGCUAUGA CGCUUAACUG
GUCUGAGAGG AUGAUCAGUC ACACUGGAAC UGAGACACGG UCCAGACUCU
ACGGGAGGCA GCAGUAGGGA AUAUUGCGCA AUGGGGGAAA CCCUGACGCA
GCAACGCCGC GUGGAGGAUG ACACUUUUCG GAGCGUAAAC UCCUUUUCUU
AGGGAAGAAU UCUGACGGUA CCUAAGGAAU AAGCACCGGC UAACUCCGUG
CCAGCAGCCG CGGUAAUACG GAGGGUGCAA GCGUUACUCG GAAUCACUGG
GCGUAAAGGG CGCGUAGGCG GAUUAUCAAG UCUCAAGUGA AAUCUAAUGG
CUUAGCCAUU AAACUGCUUG GGAAACUGAU AGUCUAGAGU GCAGGGAGAG
GCAGAUGGAU UGGUGGUGUA GGGUAAAUCC UAGAUAUCAC AGAUACAUUG
CGAGGCGAUC UCUGGAUCCA UCGAGCCUA
 oder zu einer Folge von 15 oder mehr Basen der Folge

ACCAAGAAUA CCCAUUGCGA
AGGCGAUCUG CUGGAACUCA ACUGACGCUA AGGCGCGAAA GCGUCCCCAG
CAAACAGGAU UAAGAUACCC UUGUAGUCCA CGCCCUAAAA CGACGUACAC
UAGUUAUUCC CCUGCUAGUC AUCUCUGUAU UGUCGCUAAC GCAUUAAGUG
UACCGCCUAG GGUGUACGGU CGCAAGAUUA AUUUCCGCAA CGAGCGCACC
CACGUAUUUG UUGCUAACGG UUCGGACCGA GACACUCUAA AUAGGCUGCC
UUCGUAAGGA GGAGGAAGGU GUGGACGACG UCAAGUCAUC AUGGCCCUUA
UGCCCAAGGC GACACACGUG CUACAAUGGC AUAUACAAUG AGACGCAAUA
CCGCGAGGUU GGGCAAUCUA UAAAUAUGUC CGGUUCGGAU UGUUCUCUGC
AACUCGAGAG CAUGAAGCCG GAAUCGCUAG UAAUCGUGGA UCAGCCAUGC
UACGGUGAAU ACGUUCCCGG GUCUUGGAAC UCACCGCCCG UCACACCAUG
GGAGUUGAUU UCACUCGAAG CCGGAAUACU AAACUAGUUA CCGUCCACAG
UGGAAUCACC GGCGCUGGGG UGAAGUCGUA ACAAGGUAAC CGUAGGAGAA
CCUGCGGUCG GAUCACCUCC U.

4. DNS-Testmedium nach einem der Ansprüche 1 bis 3,
dadurch gekennzeichnet, daß die Campylobacter-Spezies eine von C. jejuni, C. fetus subsp. jejuni, C. coli, Vibrio coli, C. fetus, C. fetus subsp. fetus, C. fetus subsp. intestinalis, C. fecalis oder C. hyointestinalis ist.

5. DNS-Testmedium nach einem der Ansprüche 1 bis 4,
dadurch gekennzeichnet, daß das Testmedium markiert ist.

6. Verfahren zum Erfassen der Anwesenheit von Campylobacter in einer Probe,
dadurch gekennzeichnet, daß es umfaßt:
- Bereitstellen wenigstens eines DNS-Testmediums, das zu rRNS einer Campylobacter-Spezies spezifisch hybridisieren kann und zu rRNS einer Spezies, die nicht zur Gattung Campylobacter gehört, nicht spezifisch hybridisieren kann,
- Kontaktieren dieses DNS-Testmediums mit Bakterien in der Probe unter Bedingungen, die das Hybridisieren zu rRNS dieser Campylobacter-Spezies in der Probe zur Bildung von hybriden Nucleinsäure-Komplexen erlauben, und
- Erfassen dieser hybriden Komplexe als Anzeige für Campylobacter in der Probe.

7. Verfahren nach Anspruch 6,
dadurch gekennzeichnet, daß das Testmedium aus den 5S-, 16S- oder 23S-Untereinheiten von Campylobacter-Ribosomal-RNA abgeleitet wird.

8. Verfahren nach Anspruch 6 oder 7,
dadurch gekennzeichnet, daß das Testmedium 15 oder mehr Basen aufweist und im wesentlichen komplementär ist zu einer Folge von 15 oder mehr Basen der Folge 
AAGCUUGCUA GCUUGCUAGA
AGUGGAUUAG UGGCGCACGG GUGAGUAAGG UAUAGUUAAU CUGCCCUACA
CAAGAGGACA ACAGUUGGAA ACGACUGCUA AUACUCUAUA CUCCUGCUUA
ACACAAGUUG ACUAGGGAAA GUUUUUCGGU GUAGGAUGAG ACUAUAUAGU
AUCAGCUAGU UGGUAAGGUA AUGGCUUACC AAGGCUAUGA CGCUUAACUG
GUCUGAGAGG AUGAUCAGUC ACACUGGAAC UGAGACACGG UCCAGACUCU
ACGGGAGGCA GCAGUAGGGA AUAUUGCGCA AUGGGGGAAA CCCUGACGCA
GCAACGCCGC GUGGAGGAUG ACACUUUUCG GAGCGUAAAC UCCUUUUCUU
AGGGAAGAAU UCUGACGGUA CCUAAGGAAU AAGCACCGGC UAACUCCGUG
CCAGCAGCCG CGGUAAUACG GAGGGUGCAA GCGUUACUCG GAAUCACUGG
GCGUAAAGGG CGCGUAGGCG GAUUAUCAAG UCUCAAGUGA AAUCUAAUGG
CUUAGCCAUU AAACUGCUUG GGAAACUGAU AGUCUAGAGU GCAGGGAGAG
GCAGAUGGAU UGGUGGUGUA GGGUAAAUCC UAGAUAUCAC AGAUACAUUG
CGAGGCGAUC UCUGGAUCCA UCGAGCCUA
 oder zu einer Folge von 15 oder mehr Basen der Folge

ACCAAGAAUA CCCAUUGCGA
AGGCGAUCUG CUGGAACUCA ACUGACGCUA AGGCGCGAAA GCGUCCCCAG
CAAACAGGAU UAAGAUACCC UUGUAGUCCA CGCCCUAAAA CGACGUACAC
UAGUUAUUCC CCUGCUAGUC AUCUCUGUAU UGUCGCUAAC GCAUUAAGUG
UACCGCCUAG GGUGUACGGU CGCAAGAUUA AUUUCCGCAA CGAGCGCACC
CACGUAUUUG UUGCUAACGG UUCGGACCGA GACACUCUAA AUAGGCUGCC
UUCGUAAGGA GGAGGAAGGU GUGGACGACG UCAAGUCAUC AUGGCCCUUA
UGCCCAAGGC GACACACGUG CUACAAUGGC AUAUACAAUG AGACGCAAUA
CCGCGAGGUU GGGCAAUCUA UAAAUAUGUC CGGUUCGGAU UGUUCUCUGC
AACUCGAGAG CAUGAAGCCG GAAUCGCUAG UAAUCGUGGA UCAGCCAUGC
UACGGUGAAU ACGUUCCCGG GUCUUGGAAC UCACCGCCCG UCACACCAUG
GGAGUUGAUU UCACUCGAAG CCGGAAUACU AAACUAGUUA CCGUCCACAG
UGGAAUCACC GGCGCUGGGG UGAAGUCGUA ACAAGGUAAC CGUAGGAGAA
CCUGCGGUCG GAUCACCUCC U.

9. Verfahren nach einem der Ansprüche 6 bis 8,
dadurch gekennzeichnet, daß die Campylobacter-Spezies eine von C. jejuni, C. fetus subsp. jejuni, C. coli, Vibrio coli, C. fetus, C. fetus subsp. fetus, C. fetus subsp. intestinalis, C. fecalis oder C. hyointestinalis ist.

10. Verfahren nach einem der Ansprüche 6 bis 9,
dadurch gekennzeichnet, daß die Probe eine Magenprobe, eine Stuhlprobe oder eine Nahrungsmittelprobe ist.

## Revendications

1. Une sonde d'ADN comprenant une séquence d'ADN caractérisée en ce que la séquence d'ADN est capable de s'hybrider spécifiquement à l'ARNr d'une espèce de Campylobacter, et n'est pas capable de s'hybrider spécifiquement à l'ARNr d'une espèce quelconque de bactéries non-Campylobacter.

2. Une sonde d'ADN comme revendiquée dans la revendication 1 dans laquelle ledit ARNr comprend l'une des sous-unités 5S, 16S, ou 23S des ribosomes de ladite espèce de Campylobacter.

3. Une sonde comme revendiquée dans la revendication 1 ou 2, dans laquelle la sonde comporte 15 bases ou plus et est en grande partie complémentaire d'une séquence de 15 bases ou plus de la séquence 
AAGCUUGCUA GCUUGCUAGA
AGUGGAUUAG UGGCGCACGG GUGAGUAAGG UAUAGUUAAU CUGCCCUACA
CAAGAGGACA ACAGUUGGAA ACGACUGCUA AUACUCUAUA CUCCUGCUUA
ACACAAGUUG ACUAGGGAAA GUUUUUCGGU GUAGGAUGAG ACUAUAUAGU
AUCAGCUAGU UGGUAAGGUA AUGGCUUACC AAGGCUAUGA CGCUUAACUG
GUCUGAGAGG AUGAUCAGUC ACACUGGAAC UGAGACACGG UCCAGACUCU
ACGGGAGGCA GCAGUAGGGA AUAUUGCGCA AUGGGGGAAA CCCUGACGCA
GCAACGCCGC GUGGAGGAUG ACACUUUUCG GAGCGUAAAC UCCUUUUCUU
AGGGAAGAAU UCUGACGGUA CCUAAGGAAU AAGCACCGGC UAACUCCGUG
CCAGCAGCCG CGGUAAUACG GAGGGUGCAA GCGUUACUCG GAAUCACUGG
GCGUAAAGGG CGCGUAGGCG GAUUAUCAAG UCUCAAGUGA AAUCUAAUGG
CUUAGCCAUU AAACUGCUUG GGAAACUGAU AGUCUAGAGU GCAGGGAGAG
GCAGAUGGAU UGGUGGUGUA GGGUAAAUCC UAGAUAUCAC AGAUACAUUG
CGAGGCGAUC UCUGGAUCCA UCGAGCCUA
 ou d'une séquence de 15 bases ou plus de la séquence

ACCAAGAAUA CCCAUUGCGA
AGGCGAUCUG CUGGAACUCA ACUGACGCUA AGGCGCGAAA GCGUCCCCAG
CAAACAGGAU UAAGAUACCC UUGUAGUCCA CGCCCUAAAA CGACGUACAC
UAGUUAUUCC CCUGCUAGUC AUCUCUGUAU UGUCGCUAAC GCAUUAAGUG
UACCGCCUAG GGUGUACGGU CGCAAGAUUA AUUUCCGCAA CGAGCGCACC
CACGUAUUUG UUGCUAACGG UUCGGACCGA GACACUCUAA AUAGGCUGCC
UUCGUAAGGA GGAGGAAGGU GUGGACGACG UCAAGUCAUC AUGGCCCUUA
UGCCCAAGGC GACACACGUG CUACAAUGGC AUAUACAAUG AGACGCAAUA
CCGCGAGGUU GGGCAAUCUA UAAAUAUGUC CGGUUCGGAU UGUUCUCUGC
AACUCGAGAG CAUGAAGCCG GAAUCGCUAG UAAUCGUGGA UCAGCCAUGC
UACGGUGAAU ACGUUCCCGG GUCUUGGAAC UCACCGCCCG UCACACCAUG
GGAGUUGAUU UCACUCGAAG CCGGAAUACU AAACUAGUUA CCGUCCACAG
UGGAAUCACC GGCGCUGGGG UGAAGUCGUA ACAAGGUAAC CGUAGGAGAA
CCUGCGGUCG GAUCACCUCC U.

4. Une sonde d'ADN comme revendiquée dans l'une quelconque des revendications 1 à 3 dans laquelle ladite espèce de Campylobacter est l'une des espèces C. jejuni, C. fetus subsp. jejuni, C. coli, Vibrio coli, C. fetus, C. fetus subsp. fetus, C. fetus subsp. intestinalis, C. fecalis, ou C. hyointestinalis.

5. Une sonde d'ADN comme revendiquée dans l'une quelconque des revendications 1 à 4 dans laquelle ladite sonde est marquée.

6. Une méthode pour détecter la présence de Campylobacter dans un échantillon comprenant:
la fourniture d'au moins une sonde d'ADN qui est capable de s'hybrider sélectivement spécifiquement à l ARNr d'une espèce de Campylobacter mais pas à l'ARNr d'une espèce qui n'appartient pas au genre Campylobacter,
la mise en contact de ladite sonde d'ADN avec les bactéries dans ledit échantillon dans des conditions qui permettent à ladite sonde de s'hybrider à l'ARNr de ladite espèce de Campylobacter dans ledit échantillon pour former des complexes d'acides nucléiques hybrides, et
la détection desdits complexes d'hybrides en tant qu'indication de présence de Campylobacter dans ledit échantillon.

7. Une méthode comme revendiquée dans la revendication 6 dans laquelle ladite sonde est dérivée des sous-unités 5S, 16S, ou 23S de l'ARN ribosomique de Campylobacter.

8. Une méthode comme revendiqué dans la revendication 6 ou la revendication 7 dans laquelle ladite sonde comporte 15 bases ou plus et est en grande partie complémentaire d'une séquence de 15 bases ou plus de la séquence 
AAGCUUGCUA GCUUGCUAGA
AGUGGAUUAG UGGCGCACGG GUGAGUAAGG UAUAGUUAAU CUGCCCUACA
CAAGAGGACA ACAGUUGGAA ACGACUGCUA AUACUCUAUA CUCCUGCUUA
ACACAAGUUG ACUAGGGAAA GUUUUUCGGU GUAGGAUGAG ACUAUAUAGU
AUCAGCUAGU UGGUAAGGUA AUGGCUUACC AAGGCUAUGA CGCUUAACUG
GUCUGAGAGG AUGAUCAGUC ACACUGGAAC UGAGACACGG UCCAGACUCU
ACGGGAGGCA GCAGUAGGGA AUAUUGCGCA AUGGGGGAAA CCCUGACGCA
GCAACGCCGC GUGGAGGAUG ACACUUUUCG GAGCGUAAAC UCCUUUUCUU
AGGGAAGAAU UCUGACGGUA CCUAAGGAAU AAGCACCGGC UAACUCCGUG
CCAGCAGCCG CGGUAAUACG GAGGGUGCAA GCGUUACUCG GAAUCACUGG
GCGUAAAGGG CGCGUAGGCG GAUUAUCAAG UCUCAAGUGA AAUCUAAUGG
CUUAGCCAUU AAACUGCUUG GGAAACUGAU AGUCUAGAGU GCAGGGAGAG
GCAGAUGGAU UGGUGGUGUA GGGUAAAUCC UAGAUAUCAC AGAUACAUUG
CGAGGCGAUC UCUGGAUCCA UCGAGCCUA
 ou d'une séquence de 15 bases ou plus de la séquence

ACCAAGAAUA CCCAUUGCGA
AGGCGAUCUG CUGGAACUCA ACUGACGCUA AGGCGCGAAA GCGUCCCCAG
CAAACAGGAU UAAGAUACCC UUGUAGUCCA CGCCCUAAAA CGACGUACAC
UAGUUAUUCC CCUGCUAGUC AUCUCUGUAU UGUCGCUAAC GCAUUAAGUG
UACCGCCUAG GGUGUACGGU CGCAAGAUUA AUUUCCGCAA CGAGCGCACC
CACGUAUUUG UUGCUAACGG UUCGGACCGA GACACUCUAA AUAGGCUGCC
UUCGUAAGGA GGAGGAAGGU GUGGACGACG UCAAGUCAUC AUGGCCCUUA
UGCCCAAGGC GACACACGUG CUACAAUGGC AUAUACAAUG AGACGCAAUA
CCGCGAGGUU GGGCAAUCUA UAAAUAUGUC CGGUUCGGAU UGUUCUCUGC
AACUCGAGAG CAUGAAGCCG GAAUCGCUAG UAAUCGUGGA UCAGCCAUGC
UACGGUGAAU ACGUUCCCGG GUCUUGGAAC UCACCGCCCG UCACACCAUG
GGAGUUGAUU UCACUCGAAG CCGGAAUACU AAACUAGUUA CCGUCCACAG
UGGAAUCACC GGCGCUGGGG UGAAGUCGUA ACAAGGUAAC CGUAGGAGAA
CCUGCGGUCG GAUCACCUCC U.

9. Une méthode comme revendiquée dans l'une quelconque des revendications 6 à 8 dans laquelle ladite espèce de Campylobacter est l'une des espèces C. jejuni, C. fetus subsp. jejuni, C. coli, Vibrio coli, C. fetus, C. fetus subsp. fetus, C. fetus subsp. intestinalis, C. fecalis, ou C. hyointestinalis.

10. Une méthode comme revendiquée dans l'une quelconque des revendications 6 à 9 dans laquelle ledit échantillon est un échantillon gastro-intestinal, un échantillon de selles ou un échantillon d'aliment.
